(19) Europäisches Patentamt
European Patent Office
Office européen des brevets

(11) Veröffentlichungsnummer: **0 117 447**
**B1**

(12) # EUROPÄISCHE PATENTSCHRIFT

(45) Veröffentlichungstag der Patentschrift: **09.12.87**

(51) Int. Cl.⁴: **C 07 K 7/64**, A 61 K 37/02

(21) Anmeldenummer: **84100934.3**

(22) Anmeldetag: **31.01.84**

(54) **Cyclische Peptide mit Somatostatin-Wirkung.**

(30) Priorität: **02.02.83 DE 3303345**

(43) Veröffentlichungstag der Anmeldung: **05.09.84 Patentblatt 84/36**

(45) Bekanntmachung des Hinweises auf die Patenterteilung: **09.12.87 Patentblatt 87/50**

(84) Benannte Vertragsstaaten: **AT BE CH DE FR GB IT LI LU NL SE**

(56) Entgegenhaltungen:
**EP - A - 0 029 310**
**EP - A - 0 063 308**

**NATURE, vol. 292, 1981, D.F. VEBER et al. "A potent cyclic hexapeptide analogue of somatostatin" Seiten 55-58**

(73) Patentinhaber: **HOECHST AKTIENGESELLSCHAFT, Postfach 80 03 20, D-6230 Frankfurt am Main 80 (DE)**

(72) Erfinder: **Friedrich, Axel, Dr., Johannesallee 14, D-6230 Frankfurt am Main 80 (DE)**
Erfinder: **König, Wolfgang, Dr., Eppsteiner Strasse 25, D-6238 Hofheim am Taunus (DE)**
Erfinder: **Teetz, Volker, Dr., An der Tann 20, D-6238 Hofheim am Taunus (DE)**
Erfinder: **Geiger, Rolf, Prof. Dr., Heinrich-Bleicher-Strasse 33, D-6000 Frankfurt am Main 50 (DE)**
Erfinder: **Sandow, Jürgen Kurt, Dr., Am Haideplacken 22, D-6240 Königstein/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 117 447 B1

ACTORUM AG

## Beschreibung

Somatostatin ist ein Peptid aus 14 Aminosäuren der Formel I

H-Ala-Gly-Cys-Lys-Asn-Phe-Phe-Trp-Lys-Thr-Phe-Thr-Ser-Cys-OH (I)

Es wurde sowohl im Hypothalamus (Science 179, 77–79, 1973) als auch im gastrointestinalen Trakt, wie z.B. in den D-Zellen der Pankreasinseln (Acta Physiol. Scand. Suppl. 473, 15, 1979), gefunden. Somatostatin reguliert z.B. durch Hemmung von Insulin oder Glukagon den Blutzuckerspiegel, hemmt Wachstumshormon, TSH, ACTH, Prolaktin, Pankreozymin, Sekretin, Motilin, VIP, GIP und mit Gastrin auch die Magensäuresekretion (Am. J. Med. 70, 619–626, 1981). Mit diesen Eigenschaften könnte es als Therapeutikum vielfältig verwendet werden. Über die gehemmte Ausschüttung von Insulin und Glukagon kann es bei Störungen des Blutzuckerspiegels (z.B. Diabetes) eingesetzt werden. Ein erhöhter Plasma-GH-Spiegel, der z.B. Akromegalie oder Schuppenflechte auslösen kann, wird durch Somatostatin erniedrigt. Durch die gastrinhemmende Wirkung senkt es die Magensäure und heilt die durch überschüssige Magensäure hervorgerufenen Krankheitsbilder, wie z.B. gastrointestinale Blutungen. Hormone produzierende Tumoren, die z.B. dem Verner-Morrison-Syndrom (VIPproduzierender Tumor) oder dem Zollinger-Ellison-Syndrom (gastrinproduzierender Tumor) zugrunde liegen, können mit Somatostatin im Wachstum gehemmt werden.

Somatostatin wird jedoch sehr leicht metabolisiert und kann daher sinnvoll nur als Infusion appliziert werden. Eine Suche nach stärker und länger wirksamen Somatostatinanaloga ist gerechtfertigt, um die Therapie zu vereinfachen und die Kosten zu senken. Durch die Substitution von Trp durch D-Trp wurde ein Somatostatinanalogon erhalten, das eine stärkere Wirkung zeigte. Es hemmt die Ausschüttung von Wachstumshormon und Insulin etwa 8mal und Glukagon etwa 6-mal stärker als Somatostatin (Biochem. Biophys. Res. Commun. 65, 746–51, 1975). Eine Verkürzung zu einem cyclischen Hexapeptide der Formel II

Pro-Phe-D-Trp-Lys-Thr-Phe (II)

in dem zusätzlich zu D-Trp ein Phe durch ein Pro substituiert ist, zeigt bereits eine starke und protrahierte Somatostatin-Wirkung (Nature 292, 55–58, (1981).

Es wurde nun gefunden, dass durch Substitution des Prolins in Formel II durch lipophilere Heterocyclen die Somatostatin-Wirkung weiter verstärkt werden kann.

Die Erfindung betrifft cyclische Hexa-Peptide der allgemeinen Formel III,

X-Phe-D-Trp-Lys-Y-Phe (III)

in welcher X für den Rest einer L-Aminosäure der allgemeinen Formel IIIa steht,

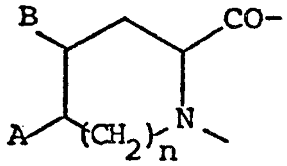

(IIIa)

wobei A und B gleich oder verschieden sind und Alkyl mit 1 bis 3 C-Atomen bedeuten oder A und B gemeinsam für eine gesättigte, ungesättigte oder aromatische mono- oder bicyclische Struktur mit 3 bis 6 C-Atomen stehen, n = 0 oder 1 bedeutet und Y für den Rest von L-Alanin, L-Serin, L-Threonin, L-Valin, L-Leucin, L-Isoleucin, L-Phenylalanin oder L-Tyrosin steht, sowie deren Salze mit physiologisch verträglichen Säuren.

Die Erfindung betrifft weiterhin ein Verfahren zur Herstellung dieser Verbindungen, das dadurch gekennzeichnet ist, dass man lineare Hexapeptide der allgemeinen Formel IV,

a-X-a-Phe-a-D-Trp-a-Lys($R^1$)-a-Y-a-Phe (IV)

in welcher X und Y die oben genannten Bedeutungen haben, $R^1$ für eine Schutzgruppe der ε-Aminofunktion steht und worin fünf der Reste a eine chemische Bindung bedeuten und einer der Reste a für -OH+ H- steht, nach bekannten Verfahren der Peptidsynthese cyclisiert und anschliessend vorhandene Schutzgruppen in geeigneter Weise abspaltet.

Unter den oben genannten Peptiden der Formel IV werden im einzelnen die nachstehenden Verbindungen der Formeln IVa–IVf verstanden,

| | |
|---|---|
| H-X-Phe-D-Trp-Lys($R^1$)-Y-Phe-OH | (IV a) |
| H-Phe-X-Phe-D-Trp-Lys($R^1$)-Y-OH | (IV b) |
| H-Y-Phe-X-Phe-D-Trp-Lys($R^1$)-OH | (IV c) |
| H-Lys($R^1$)-Y-Phe-X-Phe-D-Trp-OH | (IV d) |
| H-D-Trp-Lys($R^1$)-Y-Phe-X-Phe-OH | (IV e) |
| H-Phe-D-Trp-Lys($R^1$)-Y-Phe-X-OH | (IV f) |

in denen $R^1$, X und Y die oben genannten Bedeutungen haben.

Die Erfindung betrifft auch lineare Hexapeptide der allgemeinen Formel IV, in denen a, X, Y und $R^1$ die oben genannten Bedeutungen haben, sowie ein Verfahren zu deren Herstellung, das dadurch gekennzeichnet ist, dass man Ester der Formel IV, in welcher a $R^1$, X und Y die oben genannten Bedeutungen haben und worin fünf der Reste a eine chemische Bindung bedeuten und einer der Reste a für –OR+Z– steht, wobei R Alkyl mit 1–6 C-Atomen, vorzugsweise Methyl, bedeutet, alkalisch verseift und anschliessend den Z-Rest abhydriert.

Unter den Estern der Formel IV werden im einzelnen die Verbindungen der nachstehenden Formeln V a – V f verstanden,

Z-X-Phe-D-Trp-Lys($R^1$)-Y-Phe-OR (V a)
Z-Phe-X-Phe-D-Trp-Lys($R^1$)-Y-OR (V b)
Z-Y-Phe-X-Phe-D-Trp-Lys($R^1$)-OR (Vc)
Z-Lys($R^1$)-Y-Phe-X-Phe-D-Trp-OR (V d)
Z-D-Trp-Lys($R^1$)-Y-Phe-X-Phe-OR (V e)
Z-Phe-D-Trp-Lys($R^1$)-Y-Phe-X-OR (V f)

in denen X, Y, R und $R^1$ die oben genannten Bedeutungen haben. $R^1$ steht vorzugsweise für Boc.

Die Synthese von Verbindungen der Formeln Va – V f können sowohl mit Hilfe der Festkörpermethode nach Merrifield als auch auf klassischem Weg in Lösung durchgeführt werden. Standardverfahren sind beispielsweise in «The Peptides Analysis, Synthesis, Biology, Vol 1 Major Methods of Peptide Bond Formation, Part A», ed. E. Gross, J. Meierhofer Academic Press N.J. (1979) beschrieben.

Bei einem Cyclohexapeptid gibt es sechs Möglichkeiten um verschiedene Kettenpeptide zum gleichen Cyclopeptid zu cyclisieren. Da Tryptophan während einer sauren Schutzgruppenabspaltung (z.B. Abspaltung der Boc-Gruppe) bei der Festkörpermethode zu Nebenprodukten neigt, ist es in solchen Fällen zweckmässig, Tryptophan als letzte Aminosäure aufzusetzen. Nach der Festkörpermethode werden Hexa-Peptide hergestellt, die beispielsweise die allgemeine Formel VI

H-D-Trp-Lys(Z)-Y-Phe-X-Phe-O-$R^2$ (VI)

in der X und Y die obige Bedeutung haben und $R^2$ für das Festkörperharz steht, besitzen. Als Schutzgruppe für die ε-Aminofunktion des Lysins dient eine Urethanschutzgruppe vorzugsweise der Benzyloxycarbonylrest (Z). Die β-Hydroxygruppe des Threonins kann frei bleiben.

Das über eine Esterfunktion am Harz gebundene Hexapeptid wird mit Hydrazin abgespalten. Es entsteht das entsprechende Hydrazid, das vorzugsweise nach Überführung in das Azid cyclisiert wird. Die geschützten cyclischen Peptide werden chromatographisch gereinigt, wobei auch vorhandene Diastereoisomere getrennt werden. Nach Abspaltung der Schutzgruppen vom Benzyltyp durch katalytische Hydrierung erhält man die erfindungsgemässen Cyclopeptide.

Bei der klassischen Peptidsynthese dient beispielsweise der durch katalytische Hydrierung abspaltbare Z-Rest oder der durch sekundäre Amine abspaltbare 9-Fluorenylmethyloxycarbonylrest (Fmoc) als Aminoschutzgruppe, während die ε-Aminogruppe des Lysins vorzugsweise durch den Boc-Rest geschützt wird. Stufenweise werden Peptide der allgemeinen Formel V a-f aufgebaut.

Durch alkalische Verseifung der Ester (vorzugsweise OMe) entstehen die freien Säuren. Anschliessend wird die α-Aminoschutzgruppe abgespalten, und die am N- und C-terminalen Ende freien Peptide werden nach den Methoden der Peptidchemie cyclisiert. Die geschützten cyclischen Peptide werden chromatographisch gereinigt. Tert.-Butyl-Schutzgruppen werden vorzugsweise mittels Trifluoressigsäure, der man 1.2-Dimercaptoäthan zusetzt, abgespalten.

Racemische Aminosäuren der Formel III b

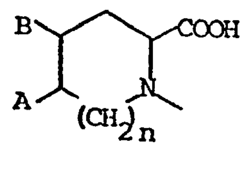

(IIIb)

in der A, B und n obige Bedeutung haben, sind beispielsweise bekannt aus der EP-A 50 800, der EP-A 31 741, der EP-A 51 020, der EP-A 49 658, der EP-A 49 605, der EP-A 29 488, der EP-A 46 953 und der EP-A 52 870. Die Tetrahydroisochinolin-3-carbonsäure ist in J. Amer. Chem. Soc. 70 (1948) 182 beschrieben. Decahydroisochinolin-3-carbonsäure ist aus der EP-A 52 870, 2,3-Dihydro-[1H]-indol-2-carbonsäure aus der US-PS 4 303 583 bekannt. Cis-exo-Octahydro-[1H]-indol-2-carbonsäure, cis, exo-Octahydro-2-cyclopenta[b]pyrrol-2-carbonsäure und cis, exo-Azabicyclo [5.3.0]-decan-3-carbonsäure sind u.a. Gegenstand der deutschen Patentanmeldung P 3 151 690.4. Die deutsche Patentanmeldung P 3 226 768.1 betrifft u.a. cis-endo-Octahydro-cyclopenta[b]pyrrol-2-carbonsäure, P 3 246 503.3 betrifft u.a cis, endo-Azabicyclo[5.3.0]decan-3-carbonsäure, P 3 210 496.0 betrifft u.a. cis, endo- und cis, exo-2,3,3a,4,5,7a-Hexahydro-[1H]indol-2-carbonsäure, P 3 300 774.8 (HOE 83/F 003) betrifft u.a. Diethylprolin und P 3 242 151.6 betrifft u.a. 3-Azatricyclo $[5.2.1.0^{2.6}]$ decan-4-carbonsäure. Aus der EP-A 37 231 ist cis, endo-Octahydro-[1H]indol-2-L-carbonsäure bekannt. Ein Verfahren zur Racematspaltung von Aminosäuren der Formel IIIb ist Gegenstand der deutschen Patentanmeldung P 3 303 112.6 (HOE 83/F 016).

Als Schutzgruppen $R^1$ sind die in M. Bodanszky et al. in «Peptide Synthesis», 2. Aufl. (1976), John Wiley & Sons beschriebenen üblichen $NH_2$-Schutzgruppen brauchbar. Bevorzugt sind Alkanoyl mit 1–6 C-Atomen, t-Butoxycarbonyl und Benzyloxycarbonyl.

Die Erfindung betrifft ferner die Verwendung der Verbindungen der Formel III als Heilmittel, pharmazeutische Präparate, die diese Verbindungen enthalten, Verfahren zu deren Herstellung und deren Verwendung als Heilmittel.

Die erfindungsgemässen Peptide haben die hemmenden Eigenschaften des Somatostatins, sie wirken jedoch wesentlich länger bei geringeren Dosen. Z.B. senken diese Peptide an der Ratte nach intravenöser Gabe die Magensäure bei einer $ED_{50}$ von ca. 5 µg/kg. Die Magensäuresenkung hält über zwei Stunden an. Zum Vergleich dazu hat Somatostatin bei einer i.v.-Dosis von 30 µg/kg keinerlei Wirkung auf die Magensäure. Erst eine Somatostatin-Infusion bewirkt eine Sen-

kung der Magensäure. Die $ED_{50}$ bei der Hemmung des Wachstumshormons liegt ebenfalls bei etwa 2–10 µg/kg i.v. Im Vergleich mit cyclo-(Pro-Phe-D-Trp-Lys-Thr-Phe) zeigte z.B. cyclo-(Aoc-Phe-D-Trp-Lys-Val-Phe) in einem Test auf die Potenzierung der blutglucosesenkenden Wirkung des Insulins bei adrenalektomierten Ratten eine mindestens doppelt so starke Wirkung. Das Aoc-Analogon senkte noch bei einer Dosierung von 0,07 $\mu g \cdot kg^{-1} \cdot min^{-1}$ signifikant die Blutglucose um bis zu 24%, während das Pro-Analogon bereits bei einer Dosierung von 0,318 $\mu g \cdot kg^{-1} \cdot min^{-1}$ völlig wirkungslos war. Auch oral und intranasal sind die neuen Verbindungen applizierbar. Wegen der schlechteren Resorbierbarkeit sind hierbei jedoch wesentlich höhere Dosen notwendig.

Wegen der somatostatinartigen Wirkung können die neuen Verbindungen überall dort eingesetzt werden, wo Somatostatininfusionen einen günstigen Effekt zeigen: z.B. bei Blutungen des gastrointestinalen Traktes, bei Magengeschwüren, bei der Behandlung von Tumoren, die durch Somatostatin hemmbare Hormone produzieren, wie z.B. beim Zollinger-Ellison-Syndrom, Verner-Morrison-Syndroms oder bei Insulin- bzw. Glukagon-produzierenden Tumoren, bei hormonabhängigen Tumoren, wenn die entsprechenden Hormone durch Somatostatin hemmbar sind, bei gewissen Leukämiearten, bei Stoffwechselstörungen mit erhöhten durch Somatostatin hemmbaren Hormonspiegeln, wie z.B. der rheumatischen Arthritis, bei der Plasma-Insulin und -Wachstumshormon zu hoch sind, bei Akromegalie, Schuppenflechte, bei Diabetes mellitus (Hemmung des Glukagons), beim Chondrosarkom und bei Schockzuständen.

Eine wirksame Dosis beim Menschen liegt bei parenteraler Applikation bei 0,1–10 µg/kg und bei intranasaler Anwendung bei etwa 1–100 µg/kg. Da exogen zugeführtes Insulin von Somatostatin nicht gehemmt wird, empfiehlt sich bei Diabetes mellitus ein Kombinationspräparat mit Insulin.

Beispiel 1

Darstellung der linearen trägergebundenen Boc-Hexapeptide der allgemeinen Formel Boc-D-Trp-Lys(Z)-Y-Phe-X-Phe-O-R.

24 g hydroxymethyliertes Merrifield-Harz schlämmt man in ca. 360 ml $CH_2Cl_2$ auf und versetzt die gerührte Suspension mit 15.9 g Boc-Phe-OH 12.4 g Dicyclohexylcarbodiimid und 7.4 g 4-Dimethylaminopyridin. Für möglichst quantitativen Umsatz lässt man über Nacht reagieren. Der feste Rückstand wird abfiltriert und gewaschen (1×50 ml $CH_2Cl_2$, 4×50 ml $CH_2Cl_2$/Methanol wie 1:1, 2×50 ml $CH_2Cl_2$). Nicht blockierte OH-Funktionen am Harz werden dann mit 3 ml Benzoylchlorid in 200 ml $CH_2Cl_2$ und 2.4 ml Pyridin als Base besetzt. Mit je 4 g dieses mit Boc-Phe-belegten Harzes führt man dann eine Festphasensynthese mit den im folgenden angegebenen Schritten durch:

| Schritt | Anzahl | Zeit (min.) | Menge (ml) | Reagenzien |
|---|---|---|---|---|
| 1 | 2 | 15 | 40 | 10% Trifluoressigsäure/0,5% Methansulfonsäure in $CH_2Cl_2$ |
| 2 | 2 | 4 | 50 | Dioxan/$CH_2Cl_2$ wie 1:1 |
| 3 | 2 | 4 | 40 | $CH_2Cl_2$/Methanol wie 1:1 |
| 4 | 3 | 3 | 50 | $CH_2Cl_2$ |
| 5 | 3 | 5 | 50 | 10% Diisopropylethylamin in $CH_2Cl_2$ |
| 6 | 5 | 3 | 50 | $CH_2Cl_2$ |
| 7 | 1 | 240 | 60 | $CH_2Cl_2$, 10 mMol Boc-Aminosäure, 10 mMol Dicyclohexylcarbodiimid, 10 mMol 1-Hydroxybenzotriazol |
| 8 | 4 | 5 | 50 | $CH_2Cl_2$/MeOH 1:1 |
| 9 | 2 | 3 | 50 | $CH_2Cl_2$ |

Die Zeitangabe für Schritt 7 wurde meist überschritten, da in der Regel über Nacht stehen gelassen werden musste. Die Kontrolle auf Vollständigkeit der Kupplung erfolgte nach Schritt 9 mit Pikrinsäure bzw. Chloranil in Toluol. Nach dem letzten Waschen der Stufe 9 wurde das beladene Merrifield-Harz scharf trockengesaugt. Ausbeute: zwischen 5,9 und 6,2 g.

Beispiel 2

Darstellung der Boc-Hexapeptid-hydrazide der allgemeinen Formel Boc-D-Trp-Lys(Z)-Y-Phe-X-Phe-$NH$-$NH_2$

6 g trägergebundenes Peptid schlämmt man in 100 ml DMF auf, versetzt mit 5 ml abs. Hydrazinhydrat und lässt 2 Tage bei Raumtemperatur rühren. Man saugt vom Rückstand ab, wäscht gut mit Dimethylformamid und Methanol und engt das Filtrat zum Trocknen ein. Zum vollständigen Entfernen überschüssigen Hydrazins versetzt man mehrfach den Rückstand mit Methanol/Toluol 1:1 und zieht am Rotationsverdampfer wieder ab. Der verbleibende Rückstand wird zur Entfernung von Benzoylhydrazid mehrfach mit wenig Wasser digeriert, schliesslich abfiltriert und im Ölpumpenvacuum über $P_2O_5$ getrocknet. Die Rohausbeuten liegen je nach Peptid zwischen 1,0 und 2,2 g.

Die rohen Peptide konnten durch Chromatographie an Kieselgel (LM: $CH_2Cl_2$/MeOH/HOAc 100:8:5) gereinigt werden.

Beispiel 3

Darstellung der Hexapeptid-hydrazide der allgemeinen Formel H-D-Trp-Lys(Z)-Y-Phe-X-Phe-$NH$-$NH_2$

1 mMol Boc-Hexapeptid-hydrazid werden in ca. 2 ml Methanol weitgehend gelöst und mit 10 ml 4N HCl in Methanol versetzt. Man lässt unter Wasserausschluss ca. 15 min rühren und engt dann am Rotavapor zur Trockne ein. Die so erhaltenen Hexapeptid-hydrazid-bis-hydrochloride setzt man ohne weitere Charakterisierung sofort in die nächste Stufe ein.

Beispiel 4

Cyclisierung zu Verbindungen der allgemeinen Formel

X-Phe-D-Trp-Lys(Z)-Y-Phe (cyclisch)

1 mMol Hexapeptid-hydrazid-dihydrochlorid werden in 25 ml DMF gelöst. Bei -15°C versetzt man die gerührte Lösung mit 0,67 ml 4 N HCl in Dioxan und anschliessend mit 0,18 ml Amylnitrit. Man lässt ca. 40 min bei tiefer Temperatur und bringt dann das Reaktionsgemisch in 2 l auf -20°C vorgekühltes DMF ein und neutralisiert mit 0,81 ml Diisopropylethylamin. Der gesamte Ansatz verbleibt 2 Tage bei ca. 0°C, dann lässt man auf Raumtemperatur kommen und rührt weitere 3 Tage nach. Der nach Abziehen der Lösungsmittel verbleibende Rückstand wird in Methanol/$H_2O$ wie 1:1 gelöst und 24 Stunden mit ca. 800 mg Mischbettionenaustauscher gerührt. Nach Abfiltrieren des Ionenaustauschers und Einengen der wässrigmethanolischen Phase verbleibt ein amorpher Rückstand, der durch semipräparative HPLC an Kieselgel (Elutionsmittel: $CH_2Cl_2$/Ethanol/Essigsäure wie 100:5:0,5) gereinigt wird.

Beispiel 5

Darstellung von Cyclopeptiden der allgemeinen Formel

X-Phe-D-Trp-Lys-Y-Phe (cyclisch)

Zur Abspaltung der ε-Z-Schutzgruppe des Lysins löst man die nach HPLC erhaltene Hauptfraktion in Methanol und hydriert ca. 2 Stunden in Anwesenheit von Palladium-Katalysator. Nach Abfiltrieren des Katalysators engt man im Vakuum ein und chromatographiert erneut an Kieselgel (Methylenchlorid/Methanol/Essigsäure/Wasser wie 70:30:1,5:6).

Tabelle 1:
Physikalische Daten für die Boc-Hexapeptidhydrazide

| X | Y | Aminosäureanalyse | | | | $[\alpha]_D^{23}$ (c = 0,5,Methanol) |
|---|---|---|---|---|---|---|
| | | Phe | Lys | X | Y | |
| Tic | Thr | 2,00 | 1,03 | 1,00 | 0,97 | -7,3° |
| Oic | Thr | 2,00 | 0,94 | 0,92 | 1,03 | -15,8° |
| Aoc | Thr | 1,99 | 0,97 | 1,00 | 1,04 | -11,1° |

Tabelle 2:
Charakteristische NMR-Daten (δ-Werte) der cyclischen Verbindung der allgemeinen Formel X-Phe-D-Trp-Lys(Z)-Y-Phe (cyclisch)

| X | Y | d, 1H Indol-NH | d, je 1H Amid-NH | d, 1H Indol-CH | d, 1H -OH | s, 2H $CH_2$(Z) | d, 3H $CH_3$(Thr) |
|---|---|---|---|---|---|---|---|
| Tic | Thr | 10,72 | 8,60<br>8,59<br>8,38<br>7,80 | 7,54 | 5,18 | 5,0 | 1,08 |
| Oic | Thr | 10,80 | 8,78<br>8,52<br>8,08 | 7,63 | | 5,0 | 0,95 |
| Aoc | Thr | 10,70 | 8,58<br>8,38<br>8,30<br>7,79 | 7,47 | | 5,0 | 1,02 |

Die Endprodukte der allgemeinen Formel

└X-Phe-D-Trp-Lys-Y-Phe┘

zeigen bis auf die fehlenden Signale für die Benzyloxycarbonyl-(Z)-Gruppe die gleichen charakteristischen NMR-Daten.

Abkürzungen:
Tic = Tetrahydroisochinolin-3-carbonsäure
Oic = cis-endo-Oktahydroindol-2-carbonsäure
Aoc = cis-Octahydro-cyclopenta[b]-pyrrol-2-endo-carbon-säure

Beispiel 6
cyclo-(Aoc-Phe-D-Trp-Lys-Val-Phe)

a) Z-Phe-Aoc-OBzl
18,5 g des Salzes aus Z-Phe-OH und H-Aoc-OBzl werden in 100 ml Dimethylformamid gelöst. Dazu gibt man bei 0°C 4,83 g HOBt und 7,0 g DCC. Man rührt eine Stunde bei 0°C und lässt über Nacht bei Raumtemperatur stehen. Der Niederschlag wird anderntags abgesaugt und das Filtrat eingeengt. Der Rückstand wird in Essigester gelöst und nacheinander mit Wasser, gesättigter, $NaHCO_3$-Lösung, 1 N $H_2SO_4$, gesättigter, $NaHCO_3$-Lösung und Wasser ausgeschüttelt. Die Essigesterlösung wird über $Na_2SO_4$ getrocknet und eingeengt.
Ausbeute: 16,1 g eines Öls.
Zur Reinigung wird an Kieselgel in Methylenchlorid/Methanol wie 9,7:0,3 (Vol.:Vol.) chromatographiert.
Ausbeute 15,4 g eines hellen Öls. DC: einheitlich in Methylenchlorid/Methanol (9,7:0,3).

b) Z-Phe-Aoc-OH
Zu einer Lösung von 15,4 g Z-Phe-Aoc-OBzl in 60 ml Dioxan/Wasser wie 8:2 gibt man 14,5 ml 2N NaOH. Man lässt etwa 20 Stunden bei Raumtemperatur stehen, neutralisiert mit 1N $H_2SO_4$ und engt ein. Der Rückstand wird mit Wasser aufgenommen und die Lösung mit 1N $H_2SO_4$ auf pH 2-3 angesäuert. Das ausfallende Öl wird mit Essigester extrahiert, die Essigesterlösung wird über $Na_2SO_4$ getrocknet und eingeengt.
Ausbeute: 13,7 g Öl.

c) Z-Phe-Aoc-Phe-OMe
Zu einer Lösung von 13,7 g Z-Phe-Aoc-OH, 6,77 g H-Phe-OMe•HCl und 4,46 g HOBt in 50 ml Dimethylformamid gibt man bei 0°C 4,02 ml N-Ethylmorpholin und 6,9 g DCC. Man lässt eine Stunde bei 0°C rühren und lässt über Nacht bei Raumtemperatur stehen. Aufarbeitung analog Beispiel 6a.
Ausbeute: 18,8 g Öl. Nach Reinigung analog 6a: 16,0 helles Öl.

d) H-Phe-Aoc-Phe-OMe•HCl
16,0 g Z-Phe-Aoc-Phe-OMe werden in Methanol gelöst. Man gibt einen 10% Pd-Kohle-Katalysator zu und hydriert am Autotitrator bei pH 4,5. Nach beendigter Hydrierung wird der Katalysator über Kieselgur abgesaugt, das Filtrat eingeengt und der Rückstand mit Diethylether verrieben. Der Niederschlag wird abgesaugt und getrocknet.
Ausbeute: 8,67 g, Schmp. 79-91°C, $[\alpha]_D^{23}$ = +26,9°C (c = 1, Methanol).

e) Z-Val-Phe-Aoc-Phe-OMe
Zu einer Lösung von 4,0 g H-Phe-Aoc-Phe-OMe•HCl, 2,016 g Z-Val-OH und 1,14 g HOBt in 30 ml Dimethylformamid gibt man bei 0°C 1,03 ml N-Ethylmorpholin und 1,76 g DC. Man lässt eine Stunde bei 0°C und anschliessend über Nacht bei Raumtemperatur stehen. Aufarbeitung und Reinigung wie bei Beispiel 6a.
Ausbeute: 4,2 g Öl.

f) H-Val-Phe-Aoc-Phe-OMe•HCl
4,95 g Z-Val-Phe-Aoc-Phe-OMe werden in Methanol gelöst und wie bei d katalytisch hydriert.
Ausbeute: 3,31 g (schmierige Substanz).

g) Z-D-Trp-Lys(Boc)-OH
Zu einer Suspension von 11,89 g H-Lys(Boc)-OH in 100 ml Dimethylformamid gibt man 6,87 g HOBt und 25 g Z-D-Trp-OTcp. Man lässt über Nacht rühren und engt ein. Der Rückstand wird dreimal mit Petrolether verrieben und in 3 Stufen zwischen Essigester und gesättigter $NaHCO_3$ gegenstromverteilt. Die Essigesterphasen werden vereinigt, mit $KHSO_4/K_2SO_4$-Lösung ausgeschüttelt, über $Na_2SO_4$ getrocknet und eingeengt.
Ausbeute: 49,1 g Öl.
Zur weiteren Reinigung wird an 400 g Kieselgel chromatographiert. Zunächst wird mit Methylenchlorid eluiert und die Substanz mit einer Mischung aus Methylenchlorid/Methanol/Wasser (Vol.:Vol.) wie 16:4:0,3 heruntergewaschen. Das Eluat wird eingeengt und am Hochvakuum getrocknet.
Ausbeute: 31,1 g amorpher Schaum.

h) Z-D-Trp-Lys(Boc)-Val-Phe-Aoc-Phe-OMe
Zu einer Lösung von 3,31 g H-Val-Phe-Aoc-Phe-OMe•HCl, 3,12 g Z-D-Trp-Lys(Boc)-OH und 0,9 g HO St in 20 ml Dimethylformamid gibt man bei 0°C 0,7 ml N-Ethylmorpholin und 1,14 g DCC. Man arbeitet analog Beispiel 6a auf.
Ausbeute: 4,16 g Schmp. 117-119°C, $[\alpha]_D^{23}$ = -24,2°C (c = 1, Methanol).

i) Z-D-Trp-Lys(Boc)-Val-Phe-Aoc-Phe-OH
2,6 g Z-D-Trp-Lys(Boc)-Val-Phe-Aoc-Phe-OMe werden in 25 ml Dioxan/Wasser (8:2) gelöst. Dazu gibt man 5 ml 1N NaOH und lässt 1,5 Stdn. bei Raumtemperatur stehen. Mit 1N $H_2SO_4$ wird neutralisiert und anschliessend die Lösung eingeengt. Der Rückstand wird analog Beispiel 6b aufgearbeitet.
Ausbeute: 2,2 g, Zers. ab 118°C, $[\alpha]_D^{23}$ = -25,9°C (c = 1, Methanol).

k) H-D-Trp-Lys(Boc)-Val-Phe-Aoc-Phe-OH
2,2 g Z-D-Trp-Lys(Boc)-Val-Phe-Aoc-Phe-OH werden in 90proz. Essigsäure gelöst und nach Zugabe eines Pd-Kohle-Katalysators hydriert. Nach

Beendigung der Hydrierung wird der Katalysator über einen Klärschichtfilter abgesaugt und das Filtrat eingeengt. Der Rückstand wird mit Wasser verrieben. Die Suspension (pH 3,1) wird unter Rühren so lange mit $NaHCO_3$-Lösung (insgesamt etwa 2,5 ml) versetzt, bis ein pH von etwa 7 erreicht ist. Der Niederschlag wird abgesaugt und gut mit Wasser gewaschen.

Ausbeute: 1,12 g, $[\alpha]_D^{23}$ = - 43,5°C (c = 1, Methanol), Zers. ab 168°C

l) cyclo-(Aoc-Phe-D-Trp-Lys(Boc)-Val-Phe

Zu einer Lösung von 481,8 mg H-D-Trp-Lys(Boc)-Val-Phe-Aoc-Phe-OH in 200 ml Dimethylformamid gibt man 0,25 ml Ethyl-methyl-phosphinsäureanhydrid (50proz.) und unter Rühren langsam eine Lösung von 0,4 ml N-Ethylmorpholin in 10 ml Dimethylformamid. Nach etwa zwei Stunden wird die Lösung eingeengt und der Rückstand mit Wasser verrieben.

Ausbeute: 0,5 g.

Zur Reinigung wird an Kieselgel in Methylenchlorid/Methanol/Wasser (1800:280:20) chromatographiert.

Ausbeute: 200 mg

m) cyclo-(Aoc-Phe-D-Trp-Lys-Val-Phe

200 mg cyclo-(Aoc-Phe-D-Trp-Lys(Boc)-Val-Phe) werden in einer Mischung aus Trifluoressigsäure/Wasser/1,2-Ethandithiol (4,5 ml:0,5 ml:0,5 ml) gelöst. Man lässt 90 Minuten bei Raumtemperatur stehen, engt ein und verteilt den Rückstand zwischen Wasser und Methyl-tert.-butylether. Die wässrige Phase wird mit einem schwach basischen Ionenaustauscher (Acetat) auf pH 3,6 gestellt und gefriergetrocknet.

Ausbeute: 126,7 mg.

Aminosäureanalyse: (Hydrolyse: 24 Stdn. bei 120°C in 6N HC1): Val (0.97), Phe (2,05), Lys (1.00), Aoc (0.95) (Gehalt an Peptidbase: 81%)

Trp wird unter diesen Bedingungen zerstört. Ein UV-Spektrum des Peptids zeigte die für Trp charakteristische Absorption bei 277 nm.

Beispiel 7

Cyclo-(D-Trp-Lys-Thr-Phe-Aoc-Phe)

a) Boc-Aoc-Phe-OBzl

9,8 g Boc-Aoc-OH, 11,2 g HCl.H-Phe-OBzl, 5,2 g HOBt und 4,9 ml NEM wurden in 100 ml DMF gelöst, mit 7,9 g DCC versetzt und 14 Stunden bei Raumtemperatur gerührt. Man saugt vom ausgefallenen DC-Harnstoff ab, dampft in Vakuum ein, nimmt den Rückstand in 200 ml Essigester auf und extrahiert mit Citronensäure- und $NaHCO_3$-Lösung. Beim Einengen der organischen Phase hinterbleibt ein öliger Rückstand.

Ausbeute: 20 g

b) H-Aoc-Phe-OBzl·TFAcO

20 g Boc-Verbindung aus Beispiel 7a werden in 50 ml Trifluoressigsäure gelöst. Nach 45 Minuten wird in Vakuum eingeengt. Man erhält wiederum ein Öl.

Ausbeute: 22 g

c) Boc-Phe-Aoc-Phe-OBzl

22 g H-Aoc-Phe-OBzl·TFAcOH, 11,5 g Boc-Phe-OH, 5,6 ml NEM und 5,9 g HOBt wurden in 150 ml Essigester gelöst. Nach Zugabe von 9,0 g DCC lässt man 18 Stunden bei Raumtemperatur reagieren. Es wurde abgesaugt, die organische Phase mit Citronensäure und $NaHCO_3$-Lösung gewaschen, über festem Natriumsulfat getrocknet, filtriert und eingeengt.

Ausbeue: 22,4 g.

d) H-Phe-Aoc-Phe-OBzl·TFAcOH

22,4 g Boc-Verbindung aus Beispiel 7c werden in 50 ml Trifluoressigsäure gelöst. Man engt nach einer Stunde bei Raumtemperatur ein.

Ausbeute: ca. 23 g

e) Z-Lys(Boc)-Thr(tBu)-Phe-Aoc-Phe-OBzl

3,3 g H-Phe-Aoc-Phe-OBzl·TFAcOH, 2,78 g Z-Lys(Boc)-Thr(tBu)-OH, 0,7 g HOBt und 0,7 ml NEM werden in 50 ml Essigester gelöst. Nach Zugabe von 1,07 g DCC lässt man bei RT über Nacht reagieren. Nach Ausschütteln mit Citronensäure und Bicarbonat-Lösung wird getrocknet und eingedampft. Das hinterbleibende braune Öl wird über 400 g Kieselgel ($SiO_2$-60, Merck AG) im System $CHCl_3$/MeOH 13:1 filtriert.

Ausbeute: 5,1 g

f) H-Lys(Boc)-Thr(tBu)-Phe-Aoc-Phe-OH

5 g Z-Verbindung (Beispiel 7e) wird in 150 ml Methanol gelöst und nach Zugabe von 0,4 g Pd/C (5% Pd) hydriert. Nach Beendigung der Wasserstoffaufnahme wird filtriert und im Vakuum eingeengt. Man chromatographiert über eine Kieselgelsäule im System $CHCl_3$/MeOH/HAcO 50:20:5.

Ausbeute: 2,1 g. Man erhält zwei Verbindungen, die Verbindung mit höherem $R_f$-Wert wird weiter umgesetzt.

g) Z-D-Trp-Lys(Boc)-Thr(tBu)-Phe-Aoc-Phe-OH

300 mg H-Lys(Boc)-Thr(tBu)-Phe-Aoc-Phe-OH, 190 mg Z-D-Trp-OTcp, 50 mg HOBt und 50 µl NEM werden in 10 ml Essigester gelöst und 48 Stunden bei Raumtemperatur belassen. Man dampft ein und chromatographiert über 150 g Kieselgel ($CHCl_3$/MeOH 5:1).

Ausbeute: 260 mg

h) H-D-Trp-Lys(Boc)-Thr(tBu)-Phe-OH

260 mg des Z-Derivates (Beispiel 7g) werden in 25 ml MeOH gelöst, mit 100 mg Pd/C versetzt und hydriert. Man filtriert den Katalysator ab und engt im Vakuum ein.

Ausbeute: ca. 240 mg

i) Cyclo-(D-Trp-Lys(Boc)-Thr(tBu)-Phe-Aoc-Phe

700 mg lineares Peptid (Beispiel 7 h) werden in 25 ml DMF gelöst und unter Rühren mit 350 µl Ethylmethylphosphinsäureanhydrid und 0,1 ml NEM versetzt. Man lässt über Nacht reagieren, dampft

im Vakuum ein und chromatographiert den Rückstand über 50 g Kieselgel im System $CH_2Cl_2$/$MeOH$/$H_2O$ 90:15:1.

Ausbeute: 500 mg

j) Cyclo-(D-Trp-Lys-Thr-Phe-Aoc-Phe)

500 mg geschütztes cyclo-Hexapeptid (Beispiel 7 i) wird in 5 ml Trifluoressigsäure gelöst. Nach 45 Minuten wird im Vakuum eingeengt und mit Ether gefällt. Man nimmt in wenig Methanol auf und fällt nochmals mit Ether aus.

Ausbeute: 450 mg

Aminosäureanalyse: Lys = 1,03; Phe = 2,0; Thr = 1,01, Aoc = 0,99;

Gehalt: 80%

Beispiel 7, Syntheseschema

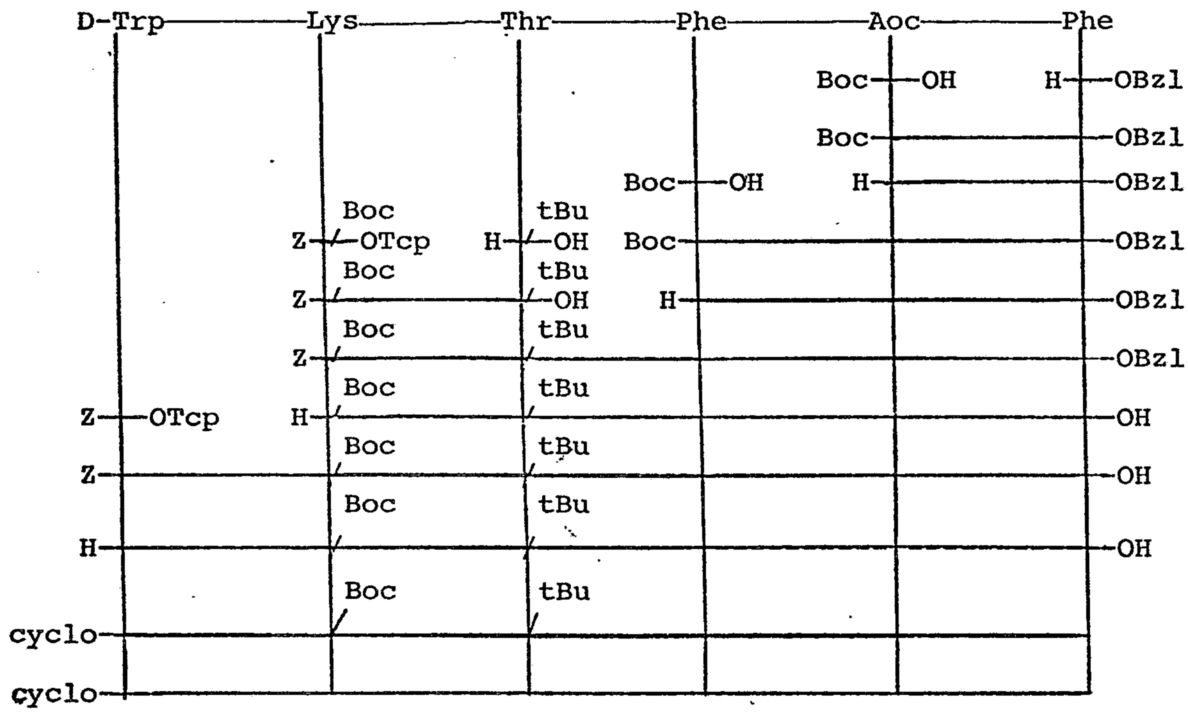


**Patentansprüche für die Vertragsstaaten BE CH DE FR GB IT LI LU NL SE**

1. Cyclisches Hexapeptid der allgemeinen Formel III

X-Phe-D-Trp-Lys-Y-Phe (III)

in welcher X für den Rest einer L-Aminosäure der allgemeinen Formel IIIa steht,

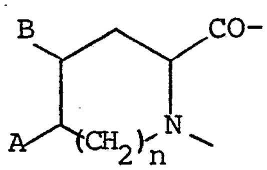

(IIIa)

wobei A und B gleich oder verschieden sind und Alkyl mit 1 bis 3 C-Atomen bedeuten oder A und B gemeinsam für eine gesättigte, ungesättigte oder aromatische mono- oder bicyclische Struktur mit 3 bis 6 C-Atomen stehen,

n = 0 oder 1 bedeutet und

Y für den Rest einer aliphatischen L-Aminosäure mit gegebenenfalls verzweigten ($C_1$-$C_4$)-Alkylseitenketten oder für den L-Phenylalanin-Rest steht, wobei die Seitenketten der genannten Reste monohydroxyliert sein können, sowie deren Salze mit physiologisch verträglichen Säuren.

2. Verbindung der Formel III gemäss Anspruch 1, in welcher Y für Thr steht.

3. Verbindung der Formel III gemäss Anspruch 1, in welcher X für den Rest der Tetrahydroisochinolin-3-L-carbonsäure steht.

4. Verbindung der Formel III gemäss Anspruch 1, in welcher X für den Rest der cis-endo-Octahydro[1H]indol-2-L-carbonsäure steht.

5. Verbindung der Formel III gemäss Anspruch 1, in welcher X für den Rest der cis, endo Octahydro-cyclopenta[b]pyrrol-2-L-carbonsäure steht.

6. Verfahren zur Herstellung einer Verbindung der Formel III, dadurch gekennzeichnet, dass man lineare Hexapeptide der allgemeinen Formel IV

a-X-a-Phe-a-D-Trp-a-Ly($R^1$)-a-Y-a-Phe (IV)

in welcher X und Y die im Anspruch 1 definierten Bedeutungen haben, $R^1$ für eine Schutzgruppe der ε-Aminofunktion steht und worin fünf der Reste a eine chemische Bindung bedeuten und einer der Reste a für -OH+H- steht, nach bekannten Verfahren der Peptidsynthese cyclisiert und anschliessend vorhandene Schutzgruppen in geeigneter Weise abspaltet und die so erhaltene Verbindung gegebenenfalls in ihr Salz überführt.

7. Hexapeptide der allgemeinen Formel IV, in der a, X, Y und $R^1$ die in Anspruch 6 definierten Bedeutungen haben.

8. Verwendung von Verbindungen der Formel II gemäss einem der Ansprüche 1-5 als Heilmittel.

9. Pharmazeutische Präparate, enthaltend eine Verbindung der Formel III gemäss einem der Ansprüche 1-5.

10. Verbindung gemäss einem der Ansprüche 1-5 zur Anwendung als Heilmittel.

**Patentansprüche für den Vertragsstaat AT**

1. Verfahren zur Herstellung eines cyclischen Hexapeptids der allgemeinen Formel III

X-Phe-D-Trp-Lys-Y-Phe (III)

in welcher X für den Rest einer L-Aminosäure der allgemeinen Formel IIIa steht,

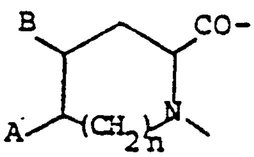

(IIIa)

wobei A und B gleich oder verschieden sind und Alkyl mit 1 bis 3 C-Atomen bedeuten oder A und B gemeinsam für eine gesättigte, ungesättigte oder aromatische mono- oder bicyclische Struktur mit 3 bis 6 C-Atomen stehen,
n = 0 oder 1 bedeutet und
Y für den Rest einer aliphatischen L-Aminosäure mit gegebenenfalls verzweigten ($C_1$-$C_4$)-Alkylseitenketten oder für den L-Phenylalanin-Rest steht, wobei die Seitenketten der genannten Reste monohydroxyliert sein können, sowie dessen Salze mit physiologisch verträglichen Säuren, dadurch gekennzeichnet, dass man lineare Hexapeptide der allgemeinen Formel IV,

a-X-a-Phe-a-D-Trp-a-Lys($R^1$)-a-Y-a-Phe (IV)

in welcher X und Y die oben genannte Bedeutung haben, $R^1$ für eine Schutzgruppe der ε-Aminofunktion steht und worin fünf der Reste a eine chemische Bindung bedeuten und einer der Reste a für -OH + H- steht, nach bekannten Verfahren der Peptidsynthese cyclisiert und anschliessend vorhandene Schutzgruppen in geeigneter Weise abspaltet und die so erhaltene Verbindung gegebenenfalls in ihr Salz überführt.

2. Verfahren gemäss Anspruch 1, dadurch gekennzeichnet, dass Y für Thr steht.

3. Verfahren gemäss Anspruch 1, dadurch gekennzeichnet, dass X für den Rest der Tetrahydroisochinolin-3-L-carbonsäure steht.

4. Verfahren gemäss Anspruch 1, dadurch gekennzeichnet, dass X für den Rest der cis-endo-Octahydro[1H]indol-2-L-carbonsäure steht.

5. Verfahren gemäss Anspruch 1, dadurch gekennzeichnet, dass X für den Rest der cis, endo-Octahydro-cyclopenta[b]-pyrrol-2-carbonsäure steht.

6. Verfahren zur Herstellung eines Hexapeptids der Formel IV, in welcher a, X, Y und $R^1$ die im Anspruch 1 definierte Bedeutung haben, dadurch gekennzeichnet, dass man Ester der Formel IV, in welcher X, Y und $R^1$ wie oben definiert sind und fünf der Reste a für eine chemische Bindung und einer der Reste a für -OR + Z stehen, wobei R Alkyl mit 1-6 C-Atomen bedeutet, alkalisch verseift und anschliessend den Z-Rest abhydriert.

7. Verfahren gemäss einem der Ansprüche 1-5 zur Herstellung einer Verbindung zur Anwendung als Heilmittel.

8. Verfahren zur Herstellung eines pharmazeutischen Präparats, enthaltend eine Verbindung der Formel III gemäss einem der Ansprüche 1-5, dadurch gekennzeichnet, dass man diese in eine geeignete Darreichungsform bringt.

**Claims for the Contracting States BE, CH, DE, FR, GB, IT, LI, LU, NL, SE**

1. A cyclic hexapeptide of the formula III

X-Phe-D-Trp-Lys-Y-Phe (III)

in which X represents the radical of an L-aminoacid of the formula IIIa

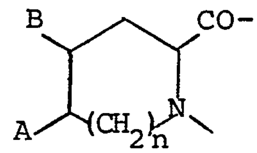

(IIIa)

in which A and B are identical or different and denote alkyl having 1 to 3 carbon atoms, or A and B together represent a saturated, unsaturated or aromatic monocyclic or bicyclic structure having 3 to 6 carbon atoms,
n denotes 0 or 1, and
Y represents an aliphatic L-aminoacid with optionally branched ($C_1$ to $C_4$)-alkyl side-chains or the radical of L-phenylalanine, the side-chains of the mentioned radicals can be monohydroxylated, and salts thereof with physiologically tolerated acids.

2. A compound of the formula III as claimed in claim 1 in which Y represents Thr.

3. A compound of the formula III as claimed in claim 1, in which X represents the radical of tetrahydroisoquinoline-3-L-carboxylic acid.

4. A compound of the formula III as claimed in claim 1, in which X represents the radical of cis,endo-octahydro-[1H]-indole-2-L-carboxylic acid.

5. The compound of the formula III as claimed in claim 1, in which X represents the radical of cis,endo-octahydrocyclopenta[b]pyrrole-2-L-carboxylic acid.

6. A process for the preparation of a compound of the formula III, which comprises cyclizing, by known processes of peptide synthesis, linear hexapeptides of the formula IV

α-X-α-Phe-α-D-Trp-α-Lys($R^1$)-α-Y-α-Phe (IV)

in which X and Y have the meanings defined in claim 1, $R^1$ represents a protective group for the ε-amino group and in which five of the moieties a denote a chemical bond and one of the moieties a represents -OH + H-, and then removing in a suitable manner protective groups which are present and, where appropriate, converting the resulting compound into its salt.

7. A hexapeptide of the formula IV in which a, X, Y and $R^1$ have the meanings defined in claim 6.

8. The use of a compound of the formula II as claimed in one of claims 1-5 as a medicine.

9. A pharmaceutical product containing a compound of the formula III as claimed in one of claims 1-5.

10. A compound as claimed in one of claims 1-5 for use as a medicine.

**Claims for the Contracting State AT**

1. A process for the preparation of a cyclic hexapeptide of the formula III

X-Phe-D-Trp-Lys-Y-Phe (III)

in which X represents the radical of an L-aminoacid of the formula IIIa

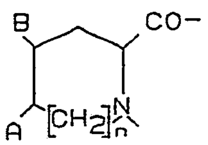

(IIIa)

in which A and B are identical or different and denote alkyl having 1 to 3 carbon atoms, or A and B together represent a saturated, unsaturated or aromatic monocyclic or bicyclic structure having 3 to 6 carbon atoms,
n denotes 0 or 1, and
Y represents an aliphatic L-aminoacid with optionally branched ($C_1$ to $C_4$)-alkyl side-chains or the radical of L-phenylalanine, the side-chains of the mentioned radicals can be monohydroxylated, and salts thereof with physiologically tolerated acids, which comprises cyclizing, by known processes of peptide synthesis, linear hexapeptides of the formula IV

α-X-α-Phe-α-D-Trp-α-Lys($R^1$)-α-Y-α-Phe (IV)

in which X and Y have the meanings defined as above, $R^1$ represents a protective group for the ε-amino group and in which five of the moieties a denote a chemical bond and one of the moieties a represents -OH + H-, and then removing in a suitable manner protective groups which are present and, where appropriate, converting the resulting compound into its salt.

2. A process as claimed in claim 1 in which Y represents Thr.

3. A process as claimed in claim 1, in which X represents the radical of tetrahydro-isoquinoline-3-L-carboxylic acid.

4. A process as claimed in claim 1, in which X represents the radical of cis,endo-octahydro-[1H]-indole-2-L-carboxylic acid.

5. A process as claimed in claim 1, in which X represents the radical of cis,endo-octahydrocyclopenta[b]pyrrole-2-L-carboxylic acid.

6. A process for the preparation of a hexapeptide of the formula IV, in which a, X, Y and $R^1$ have the meanings defined in claim 1, which comprises, alkaline hydrolysis of the esters of the formula IV in which X, Y and $R^1$ have the abovementioned meanings and in which five of the moieties a denote a chemical bond and one of the moieties a represents -OR + Z, R denoting alkyl having 1-6 carbon atoms and then removing the Z radical by hydrogenation.

7. A process as claimed in one of the claims 1-5 for the preparation of a compound for use as a medicine.

8. A process for the preparation of a pharmaceutical composition comprising a compound of the formula III as claimed in one of the claims 1-5, which comprises converting the latter into a suitable form of administration.

**Revendications pour les Etats contractants BE, CH, DE, FR, GB, IT, LI, LU, NL, SE**

1. Hexapeptide cyclique de formule générale III

X-Phe-D-Trp-Lys-Y-Phe (III)

dans laquelle X représente le reste d'un acide L-aminé de formule générale IIIa

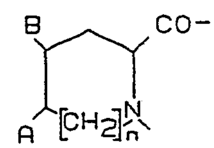

(IIIa)

A et B étant identiques ou différents et représentant un groupe alkyle ayant de 1 à 3 atomes de carbone, ou A et B formant ensemble une structure mono- ou bicyclique saturée, non saturée ou aromatique, ayant de 3 à 6 atomes de carbone;
n = 0 ou 1; et
Y représente le reste d'un acide L-aminé aliphatique à chaînes latérales alkyle en $C_1$-$C_4$ éventuellement ramifiées, ou le reste L-phénylalanine, les chaînes latérales des restes cités pouvant être monohydroxylées, et sels de celui-ci avec des acides physiologiquement acceptables.

2. Composé de formule III selon la revendication 1, dans lequel Y représente Thr.

3. Composé de formule III selon la revendication 1, dans lequel X représente le reste de l'acide tétrahydroisoquinoléine-3-L-carboxylique.

4. Composé de formule III selon la revendication 1, dans lequel X représente le reste de l'acide cis, endo-octahydro[1H]indole-2-L-carboxylique.

5. Composé de formule III selon la revendication 1, dans lequel X représente le reste de l'acide cis, endo-octa-hydro-cyclopenta[b]pyrrole-2-L-carboxylique.

6. Procédé pour la préparation d'un composé de formule III, caractérisé en ce que l'on cyclise, selon des techniques connues de la synthèse des peptides, des hexapeptides linéaires de formule générale IV

a-X-a-Phe-a-D-Trp-a-Lys($R^1$)-a-Y-a-Phe (IV)

dans laquelle X et Y ont les significations données dans la revendication 1, $R^1$ représente un groupe protecteur de la fonction ε-amino, et dans laquelle cinq des radicaux a représentent une liaison chimique et l'un des radicaux a représente -OH + H-, et on élimine ensuite de façon appropriée les groupes protecteurs présents, et on convertit éventuellement en son sel le composé ainsi obtenu.

7. Hexapeptide de formule générale IV,, dans laquelle a, X, Y et $R^1$ ont les significations données dans la revendication -6.

8. Utilisation de composés de formule II selon l'une des revendications 1à 5 comme médicaments.

9. Préparation pharmaceutique contenant un composé de formule IV selon l'une des revendications 1 à 5.

10. Composé selon l'une des revendications 1 à 5, destiné à être utilisé comme médicament.

**Revendications pour l'Etat contractant AT**

1. Procédé pour la préparation d'un hexapeptide cyclique de formule générale III

X-Phe-D-Trp-Lys-Y-Phe (III)

dans laquelle X représente le reste d'un acide L-aminé de formule générale IIIa

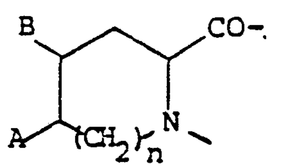

(IIIa)

A et B étant identiques ou différents et représentant un groupe alkyle ayant de 1 à 3 atomes de carbone, ou A et B formant ensemble une structure mono- ou bicyclique saturée, non saturée ou aromatique, ayant de 3 à 6 atomes de carbone;
n = 0 ou 1; et
Y représent le reste d'un acide L-aminé aliphatique à chaînes latérales alkyle en $C_1$-$C_4$ éventuellement ramifiées, ou le reste L-phénylalanine, les chaînes latérales des restes cités pouvant être monohydroxylées, ainsi que des sels de celui-ci avec des acides physiologiquement acceptables, caractérisé en ce que l'on cyclise, selon des techniques connues de la synthèse des peptides, des hexapeptides linéaires de formule générale IV

a-X-a-Phe-a-D-Trp-a-Lys($R^1$)-a-Y-a-Phe (IV)

dans laquelle X et Y ont les significations données plus haut, $R^1$ représente un groupe protecteur de la fonction ε-amino, et dans laquelle cinq des radicaux a représentent une liaison chimique et l'un des radicaux a représente -OH + H-, et on élimine ensuite de façon appropriée les groupes protecteurs présents, et on convertit éventuellement en son sel le composé ainsi obtenu.

2. Procédé selon la revendication 1, caractérisé en ce que Y représente Thr.

3. Procédé selon la revendication1, caractérisé en ce que X représente le reste de l'acide tétrahydroisoquinoléine-3-L-carboxylique.

4. Procédé selon la revendication 1, caractérisé en ce que X représente le reste de l'acide cis, endo-octahydro[1H]-indole-2-L-carboxylique.

5. Procédé selon la revendication 1, caractérisé en ce que X représente le reste de l'acide cis, endo-octahydrocyclopenta[b]pyrrole-2-carboxylique.

6. Procédé pour la préparation d'un hexapeptide de formule IV, dans laquelle a, X, Y et $R^1$ ont les significations données dans la revendication 1, caractérisé en ce que l'on saponifie par un alcali un ester de formule IV, dans laquelle X, Y et $R^1$ sont tels que définis plus haut et cinq des radicaux a représentent une liaison chimique et l'un des radicaux a représente -OR + Z, R représentant un groupe alkyle ayant de 1 à 6 atomes de carbone, et on élimine ensuite le reste Z par hydrogénation.

7. Procédé selon l'une des revendications 1 à 5 pour la préparation d'un composé à utiliser en tant que médicament.

8. Procédé pour la préparation d'une composition pharmaceutique contenant un composé de formule III selon l'une des revendicatios 1 à 5, caractérisé en ce que l'on met celui-ci sous une forme pharmaceutique appropriée.